# EUROPÄISCHE PATENTSCHRIFT

(11) **EP 2 301 502 B1**
(45) Veröffentlichungstag und Bekanntmachung des Hinweises auf die Patenterteilung: **30.04.2014**
(21) Anmeldenummer: 09012277.1
(22) Anmeldetag: 28.09.2009
(51) Int. Cl.: A61F 13/15, A61F 13/56, A61F 13/62

(54) **Verfahren zur Herstellung von Windelverschlusselementen**
Method for making nappy sealing elements
Procédé de fabrication d'éléments de fermeture de couche

(43) Veröffentlichungstag der Anmeldung: 30.03.2011
(73) Patentinhaber: Mondi Consumer Packaging Technologies GmbH, 48599 Gronau (DE)
(72) Erfinder: Bader, Herbert, 48356 Nordwalde (DE); Schönbeck, Marcus, 33775 Versmold (DE); Rolefs, Mike, 48565 Steinfurt (DE); Hagemann, Andreas, 46414 Rhede (DE); Fezert, Olga, 49809 Lingen (DE)
(74) Vertreter: Albrecht, Rainer Harald

(56) Entgegenhaltungen:
- EP-A1- 2 411 212
- US-A- 5 900 101
- US-A1- 2003 051 804
- US-B1- 6 195 850

## Beschreibung

Die Erfindung betrifft ein Verfahren zur Herstellung einer Materialbahn, aus der Windelverschlusselemente ausstanzbar sind, die einen elastisch dehnbaren Abschnitt und nichtelastische Anschlussbereiche zur Befestigung an einer Windel sowie zum Anschluss eines Verschlussteils aufweisen. Bei dem Verfahren, von dem die Erfindung ausgeht, werden parallele und zueinander beabstandete Folienstreifen aus einem elastisch dehnbaren Polymer zwischen zwei Materialbahnen aus Nonwoven einkaschiert.

Aus DE 10 2004 035 649 A1 ist ein Verfahren mit den eingangs beschriebenen Merkmalen zur Herstellung von Windelverschlusselementen bekannt, In den Bahnabschnitten zwischen den elastischen Folienstreifen werden die Nonwovenbahnen unmittelbar miteinander verklebt. Die aus der Materialbahn ausgestanzten Windelverschlusselemente weisen jeweils einen elastisch dehnbaren Abschnitt und beidseits angrenzende Anschlussbereiche aus Nonwoven auf. Die Windelverschlusselemente können als Streifen ausgebildet sein oder die Form sogenannter Windelohren aufweisen, deren Anschlussbereich an einer Windel breiter ist als der Anschlussbereich zur Befestigung des Ver schlussteils, Über das Verschlussteil werden große Kräfte auf das Windelverschlusselement übertragen. Für eine gleichmäßige Krafteinleitung in das Windelverschlusselement ist ein biegesteifer Anschlussbereich hoher Zugfestigkeit vorteilhaft. Ferner ist dafür Sorge zu tragen, dass die aus Nonwoven bestehenden Anschlussbereiche nicht ausfransen oder sich plastisch dehnen, wenn die Windelverschlusselemente in Gebrauch bis zur Dehngrenze des elastischen Bereiches gedehnt werden.

Aus US-2003/0051804 ist ein weiteres Verfahren zur Herstellung von Windelverschlusselementen bekannt.

Aus EP 1 252 015 B1 ist ein Windelverschlusselement bekannt, das eine elastische Folie als Kernschicht und beidseitig auf die Kernschicht aufkaschierte Nonwovenschichten aufweist. Die Nonwovenschichten und die Kernschicht besitzen die gleichen Außenabmessungen, d. h. die elastische Kernschicht ist in Dehnungsrichtung ebenso breit wie die beidseitig aufkaschierten Schichten aus Nonwoven. Der Anschlussbereich für das Verschlussteil ist ebenso wie der Anschlussbereich zur Befestigung an einer Windel durch eine Schicht aus einem nichtelastischen Polymer versteift. Die Versteifung erfolgt mittels einer zugfesten Folie, z. B. aus einem Polypropylen-Homopolymer, welche in den Anschlussbereichen zwischen der elastischen Folie und einer der beiden Deckschichten einkaschiert ist und die Elastizität der elastischen Kernschicht blockiert. Da elastische Polymere teure Werkstoffe sind, besteht ein Bedürfnis, den Anteil des elastischen Polymers im Verbund so gering als möglich zu halten, ohne dass sich dies nachteilig auf die Elastizität und die mechanischen Eigenschaften des Verbundmaterials auswirkt.

Aus EP 1 021 153 B1 ist ein Windelverschlusselement bekannt, das einen Träger mit elastischen und nichtelastischen Bereichen aufweist. Der Träger besteht insbesondere aus einer coextrudierten Folie mit einer elastischen Kernschicht und nichtelastischen Deckschichten. Auf einer Seite des Trägers ist ein textiles Material aufkaschiert. Auf der anderen Seite des Trägers ist ein Verschlussteil befestigt. Durch eine lokale Verstreckung, bei der bereichsweise die nichtelastischen Deckschichten des Trägers und das aufkaschierte Nonwoven überdehnt werden, wird ein elastisch dehnbarer Abschnitt erzeugt. Dies wird als selektive mechanische Aktivierung bezeichnet. Nachteilig ist auch hier, dass der elastische Träger sich über die gesamte Breite des Windelverschlusselementes einschließlich der nichtelastischen Anschlussbereiche erstreckt. Ferner ist nachteilig, dass das Windelverschlusselement nur einseitig eine textile Oberfläche aufweist.

In US 6 875 710 B2 wird ein Windelverschlusselement mit einem textilen Träger, z. B. aus Nonwoven beschrieben. In vorgegebenen Bereichen ist der Träger durch eine im thermoplastischen Zustand aufgetragene Schicht aus einem nichtelastischen Polymer verstärkt, welches die Faserstruktur der Nonwovenschicht zumindest teilweise durchsetzt. In einem anderen, davon beabstandeten Abschnitt weist der Träger eine Beschichtung aus einem thermoplastischen Elastomer auf, welche die Faserstruktur des Nonwovens ebenfalls zumindest teilweise durchsetzt und einen elastisch dehnbaren Abschnitt bildet. Die nichtelastischen und elastischen Bereiche sind räumlich zueinander beabstandet. Aufgrund des räumlichen Abstandes ist die Kraftübertragung zwischen den verstärkten bzw. versteiften nichtelastischen Bereichen und dem elastischen Bereich unbefriedigend. Wird das Material bis zur Dehngrenze des elastischen Bereiches gedehnt, besteht die Gefahr, dass das ausschließlich aus Nonwoven bestehende Material zwischen dem elastischen Bereich und den nichtelastischen Bereichen sich plastisch verformt und zerstört wird.

Der Erfindung liegt die Aufgabe zugrunde, ein kostengünstiges Verfahren zur Herstellung von Windelverschlussefementen anzugeben, die einen biegesteifen und zugfesten Anschlussbereich für ein Verschlussteil aufweisen.

Gegenstand dieser Erfindung und Lösung dieser Aufgabe ist ein Verfahren nach Anspruch 1.

Bei dem erfindungsgemäßen Verfahren werden in einem ersten Verfahrensschritt biegesteife Verstärkungsschichten aus einem nichtelastischen Polymer in parallelen und zueinander beabstandeten Streifen auf eine erste Nonwovenbahn aufgebracht. Anschließend wird die erste Nonwovenbahn in einem Kaschierwerk mit elastischen Folienstreifen und einer zweiten Nonwovenbahn zu einem Laminat verbunden, welches die Verstärkungsschichten sowie die elastischen Folienstreifen jeweils als Einlage zwischen Außenschichten aus Nonwoven enthält. Das Laminat weist vorzugsweise quer zur Bahnrichtung eine sich mehrfach wiederholende Folge aus drei Zonen auf. In einer ersten Zone sind die Nonwovenbahnen unmittelbar miteinander verbunden. Eine zweite Zone ist elastisch dehnbar und weist Außenschichten aus Nonwoven sowie einen elastischen Folienstreifen als Zwischenschicht auf. Die dritte Zone weist zwei Außenschichten aus Nonwoven und eine Verstärkungsschicht als Zwischenschicht auf. Für das erfindungsgemäße Verfahren ist wesentlich, dass die Lage der elastischen Folienstreifen und der Verstärkungsschichten so aufeinander abgestimmt werden, dass die Verstärkungsschichten den Abstand zwischen zwei elastischen Folienstreifen ausfüllen und in beidseitigen Überlappungsbereichen mit den angrenzenden elastischen Folienstreifen verbunden sind. Die Überlappungsbereiche weisen zweckmäßig jeweils eine Breite zwischen 2 mm und 10 mm auf. Hierdurch ist eine gleichmäßige Kraftübertragung zwischen dem elastisch dehnbaren Abschnitt und den angrenzenden Abschnitten gewährleistet, wenn beim Gebrauch der nach dem erfindungsgemäßen Verfahren hergestellten Windelverschlusselemente Zugkräfte auf den durch eine biegesteife Verstärkungsschicht verstärkten Anschlussbereich ausgeübt wird. Nach dem erfindungsgemäßen Verfahren können auch Windelverschlusselemente hergestellt werden, die einen verstärkten Anschlussbereich zum Anschluss eines Verschlussteils und einen weichen, flexiblen Anschlussbereich zur Befestigung an einer Windel aufweisen. Hierbei ist lediglich der Anschlussbereich zum Anschluss eines Verschlussteils mit einer der Versteifung dienenden Verstärkungsschicht verstärkt ist, wohingegen der andere Anschlussbereich zur Befestigung an einer Windel ausschließlich aus den beiden Nonwovenschichten besteht, die unmittelbar miteinander verbunden sind und einen weichen, flexiblen Anschlussbereich formen, der sich an die Kontur einer Windel gut anpassen kann.

Das Anbringen der Verstärkungsschichten an der ersten Nonwovenbahn ist auf verschiedene Weise möglich. Eine erste Ausgestaltung des erfindungsgemäßen Verfahrens sieht vor, dass die Verstärkungsschichten durch Extrusionsbeschichtung auf die erste Nonwovenbahn aufgebracht werden, wobei vorzugsweise zwischen dem nichtelastischen Polymer für die Verstärkungsschichten und der Nonwovenbahn aus den nachfolgenden Gründen noch eine perforierte Folie angeordnet wird.

Die zwischen den Nonwovenbahnen eingebrachten Verstärkungsschichten sollen zugleich eine hohe Biegesteifigkeit sowie eine hohe Weiterreißfestigkeit aufweisen. Diese beiden Anforderungen lassen sich alleine durch die Auswahl des Polymers nicht im gewünschten Maße gleichzeitig realisieren. Wählt man ein sprödes Polymer, so resultieren gute Biegesteifigkeitswerte. Allerdings nimmt die Weiterreißfestigkeit mit zunehmender Sprödigkeit des Polymers ab. Das Nonwoven, welches eine hohe Weiterreißfestigkeit aufweist, übernimmt bei vollflächigem Polymerauftrag die schlechten Weiterreißeigenschaften des Polymers. Wird ein elastisches Polymer verwendet, so kann zwar die Weiterreißfestigkeit verbessert werden, allerdings nimmt im Gegenzug die Biegesteifigkeit ab. Wenn zwischen der ersten Nonwovenbahn und der im Wege der Extrusionsbeschichtung aufgebrachten Verstärkungsschichten eine perforierte Folie als Zwischenschicht angeordnet wird, können zur Erzeugung der Verstärkungsschichten spröde Polymere eingesetzt werden, ohne dass sich dies nachteilig auf die Weiterreißfestigkeit des Laminats auswirkt, Das bei einer Extrusionsbeschichtung im schmelzeflüssigen Zustand aufgebrachte Polymer migriert durch die Öffnungen der perforierten Folie in das Nonwoven, so dass das Polymer nicht mehr vollflächig, sondern nur noch punktuell mit dem Nonwoven verbunden wird. Die Polymermasse der Verstärkungsschicht wird dadurch von dem Nonwoven entkoppelt, so dass die guten Weiterreißeigenschaften des Nonwovens zum Tragen kommen. Gemäß einer bevorzugten Ausführung des erfindungsgemäßen Verfahrens wird daher vorgesehen, dass auf die erste Nonwovenbahn zumindest im Bereich der aufzubringenden Verstärkungsschichten eine perforierte Folie aufgelegt wird und dass das nicht elastische Polymer für die Verstärkungsschichten durch eine Extrusionsbeschichtung anschließend auf die perforierte Folie aufgebracht wird, wobei das schmelzeflüssige Polymer die Perforationsöffnungen der Folie durchdringt und sich im Bereich der Perforationsöffnungen mit der Nonwovenbahn verbindet. Vorzugsweise erfolgt eine Befestigung der aufgelegten perforierten Folie erst dadurch, dass bei der Extrusionsbeschichtung das schmelzflüssige Polymer die Perforationsöffnungen durchdringt. Als perforierte Folie können Folien aus preiswerten Polymeren eingesetzt werden, da die beschriebene Funktion primär durch die Zahl und Größe der Perforationsöffnungen bestimmt wird und die mechanischen Eigenschaften der Perforationsfolie von untergeordneter Bedeutung ist. Allerdings muss die Polarität der perforierten Folie auf das bei der Extrusionsbeschichtung verwendete Polymer abgestimmt sein.

Zum Auftragen des Polymers wird vorzugsweise ein Auftragswerkzeug verwendet, welches an der zu beschichtenden Bahn anliegt, damit das Polymer für die Verstärkungsschicht in die Struktur der Bahn, d. h. in die Struktur des Nonwovens bzw. die Perforationsöffnungen einer aufkaschierten perforierten Folie, eindringt.

Der streifenförmige Polymerauftrag kann flächig in einer gleichförmigen Schichtdicke oder in Form eines Musters, welches sich aus Polymerflächen und polymerfreien Flächen zusammensetzt, ausgeführt werden. Zur Extrusionsbeschichtung kann eine Walze verwendet werden, deren Walzenoberfläche eine Struktur aus Erhebungen und/oder Vertiefungen autweist, Durch die profilierte Walzenoberfläche kann ein Polymermuster mit unterschiedlichen Auftragsmengen erzielt werden, wodurch der Versteifungseffekt beeinflusst werden kann. Außerdem bewirkt die Struktur aus Erhebungen und/oder Vertiefungen, dass das Polymer weiter in das Nonwoven der zu beschichtenden Bahn eindringt. Bei der Verwendung einer strukturierten Walze kann deshalb auch bei einem Curtain-Coating-Verfahren, bei dem das schmelzflüssige Polymer zunächst lediglich auf die zu beschichtende Bahn aufgelegt wird, eine ausreichend feste Verbindung erreicht werden.

Als Auftragswerkzeug für die Extrusionsbeschichtung kann auch eine Beschichtungsdüse mit einer Mehrzahl von Austrittsöffnungen verwendet werden, die in einem an den Abstand der Verstärkungsschichten angepassten Abstand zueinander angeordnet sind. Als Auftragswerkzeuge können ferner parallel geschaltete Beschichtungsdüsen eingesetzt werden, deren Abstand vorzugsweise einstellbar ist. Die Applikation des Polymers erfolgt im Bereich der Verstärkungsschichten jeweils flächig oder in einem Muster.

Das erfindungsgemäße Verfahren ist nicht darauf beschränkt, dass die Verstärkungsschichten im Wege der Extrusionsbeschichtung erzeugt werden. Im Rahmen der Erfindung liegt es auch, dass zur Herstellung der Verstärkungsschichten Verstärkungsstreifen aus einer biegesteifen Polymerfolie oder einem Nonwoven verwendet werden. Die Befestigung der Verstärkungsstreifen an der Nonwovenbahn erfolgt thermisch oder durch Verkleben.

Eine Ausführung des erfindungsgemäßen Verfahrens sieht vor, dass die Ver stärkungsstreifen aufgeheizt und in heißem Zustand mit der Nonwovenbahn verpresst werden. Insbesondere können die Verstärkungsstreifen mittels Prägewalzen unter Druck und Wärme mit der Nonwovenbahn verbunden werden. Eine thermische Befestigung kann dabei auch durch Ultraschallschweißen erfolgen. Im Rahmen der Erfindung liegt es auch, eine thermische Verbindung erst dann zu erzeugen, wenn die Verstärkungsstreifen zwischen der ersten und der zweiten Nonwovenbahn angeordnet sind, wobei dann die Verbindung mit beiden Nonwovenbahnen erfolgt.

Die Verstärkungsstreifen können ferner mit den beidseitigen Nonwovenbahnen verklebt werden. Dabei werden die Verklebungen vorzugsweise so ausgeführt, dass die Klebeverbindungen an den beiden Seiten der Verstärkungsstreifen unterschiedliche Haftfestigkeiten aufweisen. Eine geringe Haftfestigkeit zwischen Verstärkungsstreifen und angrenzender Nonwovenbahn an zumindest einer Seite des Laminats wirkt sich vorteilhaft auf die Weiterreißfestigkeit des Verbundes aus.

Unterschiedliche Haftfestigkeiten an den beiden Seiten einer Verstärkungsschicht können dadurch realisiert werden, dass die Verstärkungsstreifen an einer Seite durch einen flächigen Klebstoffauftrag und an der anderen Seite durch einen partiellen Klebstoffauftrag mit der jeweils angrenzender Nonwovenbahn verbunden werden. Für den partiellen Klebstoffauftrag kann insbesondere eine Wirbeldüsentechnik verwendet werden. Zur Verbesserung der Klebstoff haftung können die Verstärkungsstreifen ein oder beidseitig vorbehandelt werden. Eine Vorbehandlung an nur einer Seite der Verstärkungsstreifen kann auch genutzt werden, um gezielt unterschiedliche Klebstoffhaftungen an beiden Seiten der Verstärkungsschicht zu erzeugen.

Unabhängig davon, ob die Verstärkungsschichten aus einkaschierten biegesteifen Polymerfolien hergestellt werden oder im Wege der Extrusionsbeschichtung erzeugt werden, können die Verstärkungsschichten insbesondere aus Polyolefinen, Polyolefin-Copolymeren, Styrolpolymeren, Cycloölefin-Copolymeren, Polyamiden, einem Polyactid, Polyester, thermoplastischen Polyurethanen oder Mischungen dieser Polymere hergestellt werden.

Zur Herstellung des Verbundes zwischen den Nonwovenbahnen und den einkaschierten elastischen Folienstreifen werden vorzugsweise Hot-Melt-Klebstoffe verwendet. Nach dem Kaschierprozess durchläuft die Materialbahn ferner zweckmäßig eine Walzenanordnung aus ineinandergreifenden profilierten Streckwalzen, in der die Materialbahn im Bereich der einkaschierten elastischen Folienstreifen quer zur Bahnrichtung verstreckt wird. Durch die Verstreckung werden die elastischen Bereiche der Materialbahn aktiviert, so dass die daraus ausgestanzten Windelverschlusselemente mit geringer Kraft bis zu einer deutlich wahrnehmbaren Dehnungsgrenze gedehnt werden können.

Im Folgenden wird die Erfindung anhand von Ausführungsbeispielen näher erläutert. Es zeigen schematisch:
- **Fig.1**: einen Längsschnitt in Dehnungsrichtung durch einen nach dem erfindungsgemäßen Verfahren hergestellten Windelverschluss- element,
- **Fig. 2**: ein Verfahren zur Herstellung einer Materialbahn, aus der Windelverschlusselemente gemäß Fig. 1 ausstanzbar sind,
- **Fig. 3**: eine Draufsicht auf eine nach dem Verfahren gemäß Fig. 2 hergestellt Materialbahn,
- **Fig. 4**: das Aufbringen einer Verstärkungsschicht auf eine Nonwoven- bahn durch Extrusionsbeschichtung im Zuge der Durchführung des in Fig. 2 dargestellten Verfahrens,
- **Fig. 5A bis 5H**: verschiedene Muster des eine Verstärkungsschicht bildenden Polymerauftrags bei Durchführung des in Fig. 2 dargestellten Verfahrens,
- **Fig. 6 und 7**: weitere Verfahren zur Herstellung einer Materialbahn, aus der Windelverschlusselemente gemäß Fig. 1 ausstanzbar sind,
- **Fig. 8A bis 8C**: verschiedene Muster für eine thermische Verbindung eines zugeführten Verstärkungsstreifens bei Durchführung des in der Fig. 6 dargestellten Verfahrens.

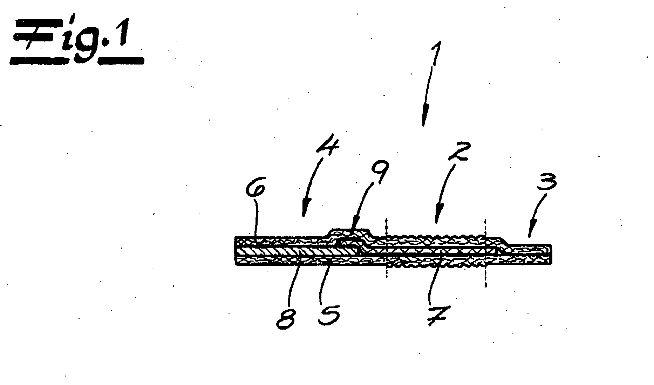

Das in Fig. 1 dargestellte Windelverschlusselement 1 weist einen elastisch dehnbaren Abschnitt 2, einen nichtelastischen Anschlussbereich 3 zur Befestigung an einer Windel sowie einen ebenfalls nichtelastischen Anschlussbereich 4 zum Anschluss eines Verschlussteils auf. Das Windelverschlusselement 1 ist mehrschichtig aufgebaut und weist Deckschichten 5, 6 aus Nonwoven sowie einen zwischen den Deckschichten 5, 6 einkaschierten elastischen Folienstreifen 7 auf. Die Deckschichten 5, 6 sind in Dehnungsrichtung breiter als der einkaschierte elastische Folienstreifen 7 und sind an überstehenden Abschnitten, welche die nichtelastischen Anschlussbereiche 3, 4 bilden, miteinander verbunden. Der Anschlussbereich 3 zur Befestigung an einer Windel ist weich und flexibel und setzt sich lediglich aus den beiden miteinander verbundenen Nonwovenschichten 5, 6 zusammen. Der Anschlussbereich 4 für das Verschlussteil hingegen ist durch eine Verstärkungsschicht 8 versteift, die in einem Überlappungsbereich 9 an dem mit der einkaschierten elastischen Folie 7 versehenen Abschnitt des Windelverschlusselementes verankert ist. Der Überlappungsbereich 9 ist etwa 2 mm bis 10 mm breit. Die Verstärkungsschicht 8 besteht aus einem Polymer, welches sich im Vergleich zu den mechanischen Eigenschaften des elastischen Folienstreifens 7 durch eine wesentlich größere Biegesteifigkeit und größere Zugfestigkeit auszeichnet. Geeignet sind Poly ethylene, Polypropylene, insbesondere Polypropylen-Homopolymere, Poly olefin-Copolymere, Stryolpolymere, Cycloolefin-Copolymere, Polyamide, Poly actid, thermoplastische Polyurethane oder Mischungen dieser Polymere.

Der elastisch dehnbare Bereich ist durch eine monoaxiale Verstreckung des die einkaschierte elastische Folie 7 enthaltenden Abschnitts des Laminats aktiviert worden. Durch eine lokal begrenzte Verstreckung werden die Fasern der Nonwovenschicht 5, 6 überdehnt und der Dehnungswiderstand in diesem Bereich reduziert. Der verstreckte Bereich ist schmaler als der elastische Folienstreifen 7 und endet vor dem Überlappungsbereich 9.

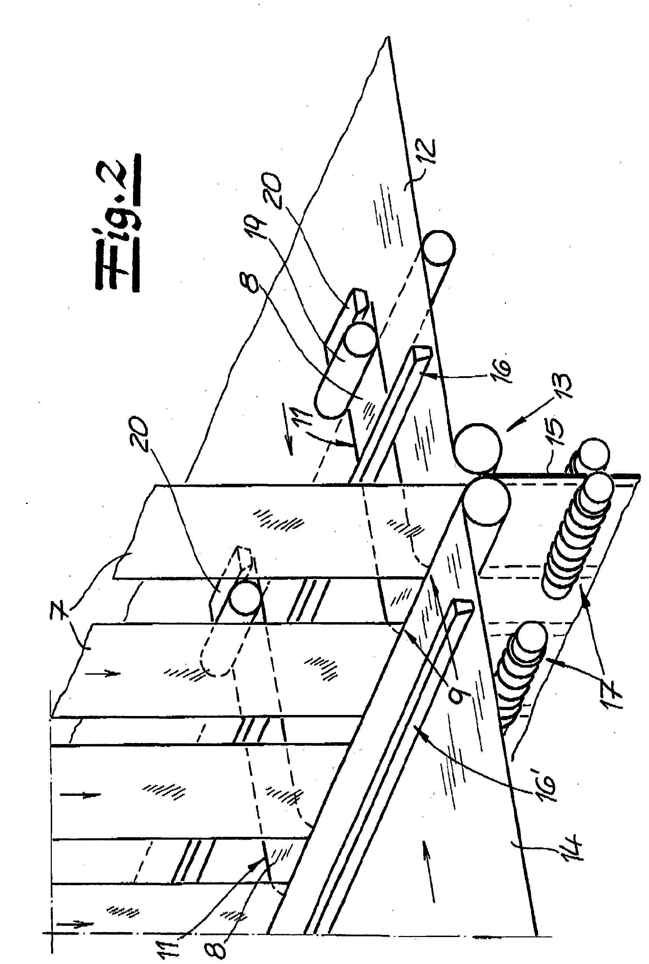

In Fig. 2 ist ein Verfahren dargestellt zur Herstellung einer Materialbahn 15, aus der Windelverschlusselemente 1 ausstanzbar sind, die den in Fig. 1 dargestellten Aufbau aufweisen. Bei dem in Fig. 2 dargestellten Verfahren werden biegesteife Verstärkungsschichten 8 aus einem nichtelastischen Polymer in parallelen und zueinander beabstandeten Streifen 11 auf eine erste Nonwovenbahn 12 aufgebracht. Anschließend wird die erste Nonwovenbahn 12 in einem Kaschierwerk 13 mit den elastischen Folienstreifen 7 und einer zweiten Nonwovenbahn 14 zu einem Laminat bzw. einer mehrlagigen Materialbahn 15 verbunden. Die Lagen des Laminats werden miteinander verklebt, wobei der Klebstoff gemäß der Darstellung in Fig. 2 beispielsweise in Stationen 16,16' auf die innenliegenden Flächen der Nonwovenbahnen 12, 14 aufgetragen wird. Der Auftrag des Klebstoffes erfolgt flächig oder in einem regelmäßigen Muster aus klebstofffreien Flächen und Klebeflächen. Dabei werden zweckmäßig Hot-Meit-Klebstoffe verwendet. Die Lage der elastischen Folienstreifen 7 und der Verstärkungsschichten 8 werden bei der Durchführung des Verfahrens so aufeinander abgestimmt, dass die Verstärkungsschichten 8 den Abstand zwischen zwei elastischen Folienstreifen 7 ausfüllen und in beidseitigen Überlappungsbereichen 9 mit den angrenzenden elastischen Folienstreifen 7 verklebt werden.

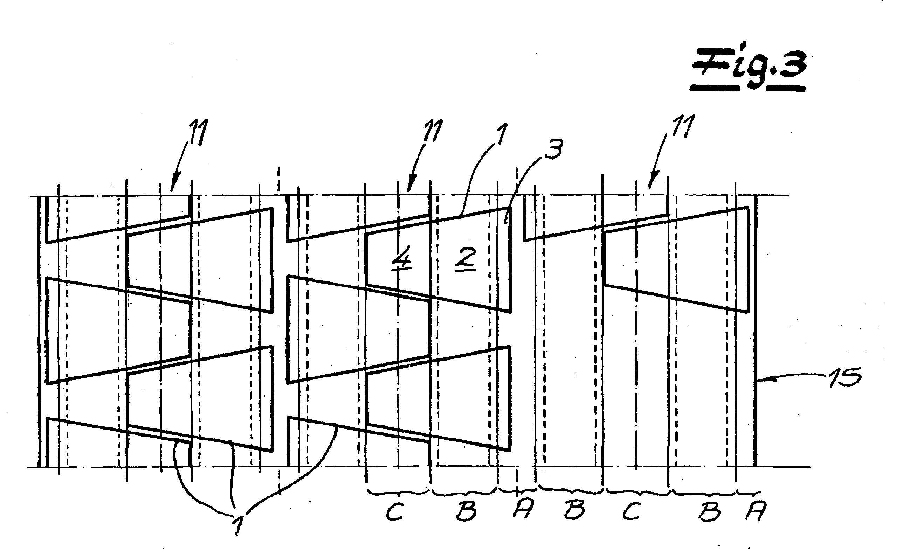

Nach dem Kaschierprozess durchläuft das Laminat 15 eine Walzenanordnung 17 aus ineinandergreifenden profilierten Streckwalzen, in der das Laminat 15 im Bereich der einkaschierten elastischen Folienstreifen 7 quer zur Bahnrichtung lokal verstreckt wird. Durch die Verstreckung erfolgt eine Aktivierung der dehnbaren Bereiche und wird in den dehnbaren Bereichen der Materialbahn 15 der Dehnungswiderstand reduziert. Die Fig. 3 zeigt eine Draufsicht auf das Laminat bzw. die mehrlagige Materialbahn 15. Das Ausführungsbeispiel zeigt eine mehmutzige Materialbahn 15, die quer zur Bahnrichtung eine sich mehrfach wiederholende Folge aus drei Zonen aufweist. In einer ersten Zone A sind die Nonwovenbahnen 12, 14 unmittelbar miteinander verbunden. Eine zweite Zone B ist elastisch dehnbar und weist Außenschichten aus Nonwoven sowie einen elastischen Folienstreifen 7 als Zwischenschicht auf. Die dritte Zone C weist von den Nonwovenbahnen 12, 14 gebildete Außenschichten sowie eine Verstärkungsschicht 8 als Zwischenschicht auf. Die mechanische Aktivierung der dehnbaren Abschnitte erzeugt eine in der Draufsicht streffenförmige Struktur, die abhängig ist von der Profilierung der Streckwalzen. Aus der Materialbahn 15 können Windelverschlusselemente 1 ausgestanzt werden, deren Kontur in Fig. 3 zur Veranschaulichung dargestellt wurde. Bei den Windelverschlusselementen 1 handelt es sich um sogenannte Windelohren, deren windelseitiger Anschlussbereich 3 in Maschinenlaufrichtung der Materialbahn 15 länger ist als der Anschlussbereich 4 zur Befestigung eines Ver schlussteils. Das Ausführungsbeispiel zeigt eine mehmutzige Materialbahn 15, die mehrere Streifen 11 der Verstärkungsschicht aufweist.

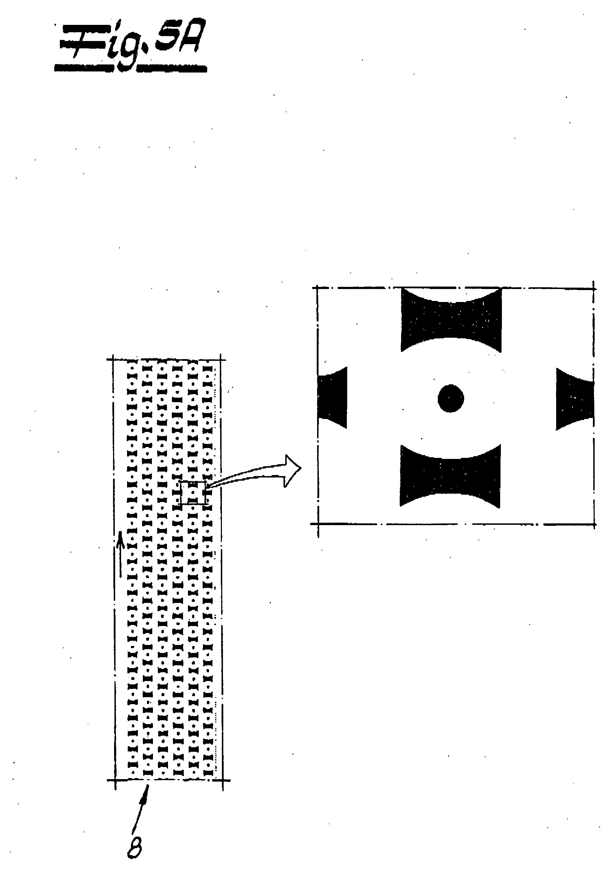

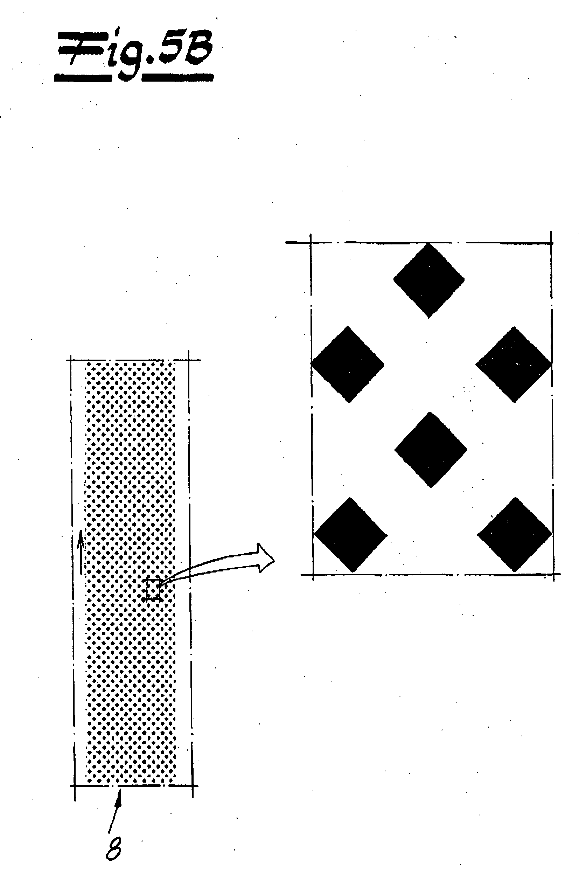

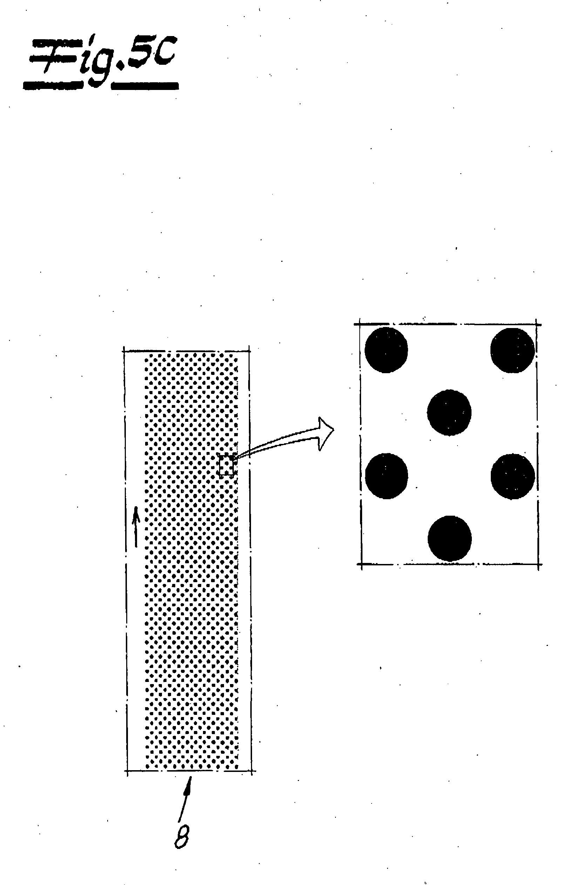

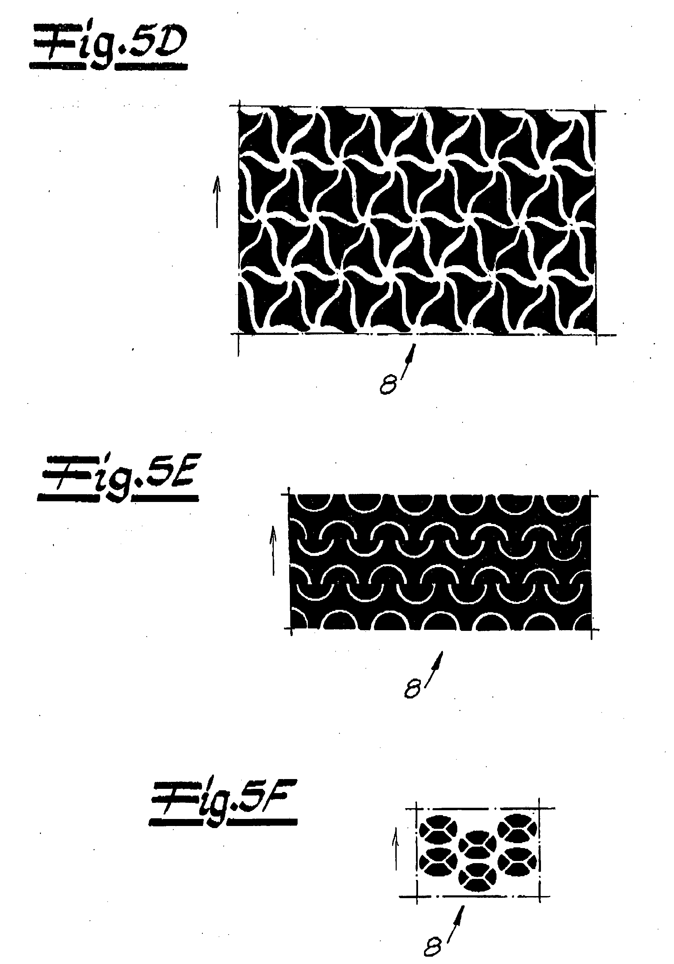

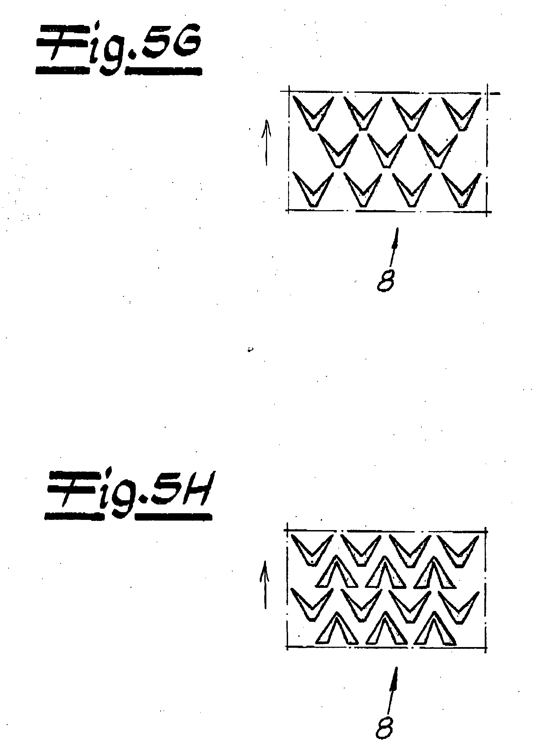

Die streifenförmig einkaschierten Verstärkungsschichten 8 können auf verschiedene Weise auf die erste Nonwovenbahn 12 aufgebracht werden. Bei dem in Fig. 2 dargestellten Verfahren werden die Verstärkungsschichten 8 durch eine Extrusionsbeschichtung auf die erste Nonwovenbahn 12 aufgebracht. Die Nonwovenbahn 12 wird über eine Walze 19 geführt, deren Oberfläche vorzugsweise eine Struktur aus Erhebungen und Vertiefungen aufweist. Zum Auftragen des Polymers wird zweckmäßig ein Auftragswerkzeug 20 verwendet, welches an der Nonwovenbahn 12 anliegt, damit das Polymer in die Struktur der Bahn eindringt. Durch eine Profilierung der Walzenoberfläche kann ein Polymer muster mit unterschiedlichen Auftragsmengen erzielt werden, wodurch der Ver steifungseffekt beeinflusst wird. Vorteilhafte Polymermuster sind in den Fig. 5A bis 5H dargestellt. Der in den Fig. 5A bis 5H dargestellte Richtungspfeil zeigt die Längserstreckung einer durch Extrusionsbeschichtung auf die Nonwovenbahn 12 aufgetragenen Verstärkungsschicht 8. Die Profilierung der Walzenoberfläche trägt ferner dazu bei, dass die Materialmenge optimal eingesetzt und im Ergebnis Material an Versteifungspolymer eingespart wird. Als Auftragswerkzeug können parallel geschaltete Beschichtungsdüsen verwendet, werden, deren Abstand vorzugsweise einstellbar ist. Alternativ kann auch eine Beschichtungsdüse mit einer Mehrzahl von Austrittsöffnungen verwendet werden, welche in einem an den Abstand der Verstärkungsschichten 8 angepassten Abstand angeordnet sind.

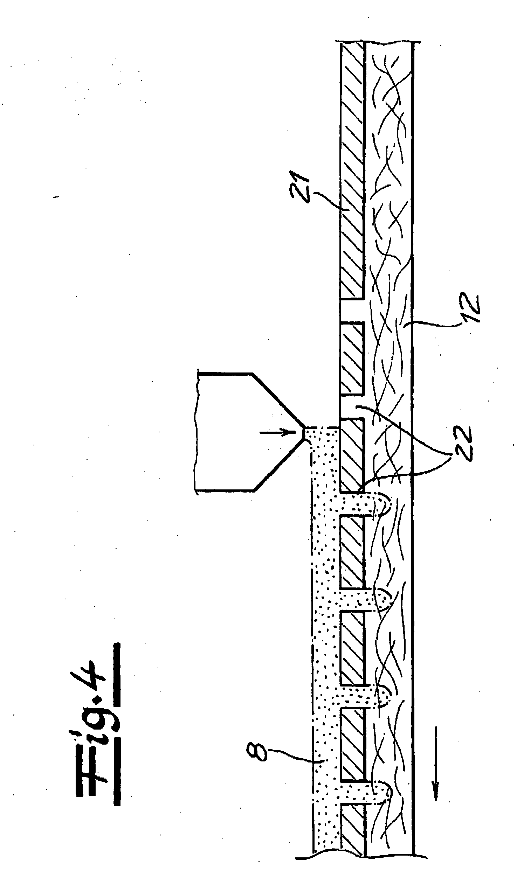

Das die Verstärkungsschicht 8 bildende Polymer kann unmittelbar auf die Nonwovenoberfläche aufgebracht werden. Gemäß einer bevorzugten Ausführung des erfindungsgemäßen Verfahrens wird jedoch zwischen der Nonwovenbahn 12 und dem Verstärkungspolymer eine perforierte Folie 21 angeordnet, wie dies in Fig. 4 dargestellt ist. Der Darstellung in Fig. 4 ist zu entnehmen, dass auf die Nonwovenbahn 12 ganzflächig oder zumindest im Bereich der aufzubringenden Verstärkungsschichten 8 eine perforierte Folie 21 aufgelegt worden ist. Das vichtelastische Polymer für die Verstärkungsschichten 8 wird durch Extrusionsbeschichtung anschließend auf die perforierte Folie 21 aufgebracht, wobei das schmelzeflüssige Polymer die Pertorationsöffnungen 22 der Folie durchdringt und sich im Bereich der Perforationsöffnungen 22 mit der Nonwovenbahn 12 verbindet. Durch die zwischengeschaftete Perforationsfolie 21 kann die Weiterreißfestigkeit des Laminats verbessert werden. Dies ist darauf zurückzuführen, dass das die Verstärkungsschichten 8 bildende Polymer, weiches zwar gute Biegesteifigkeitswerte aber zumeist nur schlechte Weiterreißeigenschaften aüfweist, nicht mehr vollflächig sondern nur noch punktuell mit der Nonwovenbahn 12 verbunden ist. Die Polymermasse der Verstärkungsschichten 8 ist somit von dem Nonwoven entkoppelt, wodurch die günstigen Weiterreißeigenschaften des Nonwovens zum Tragen kommen.

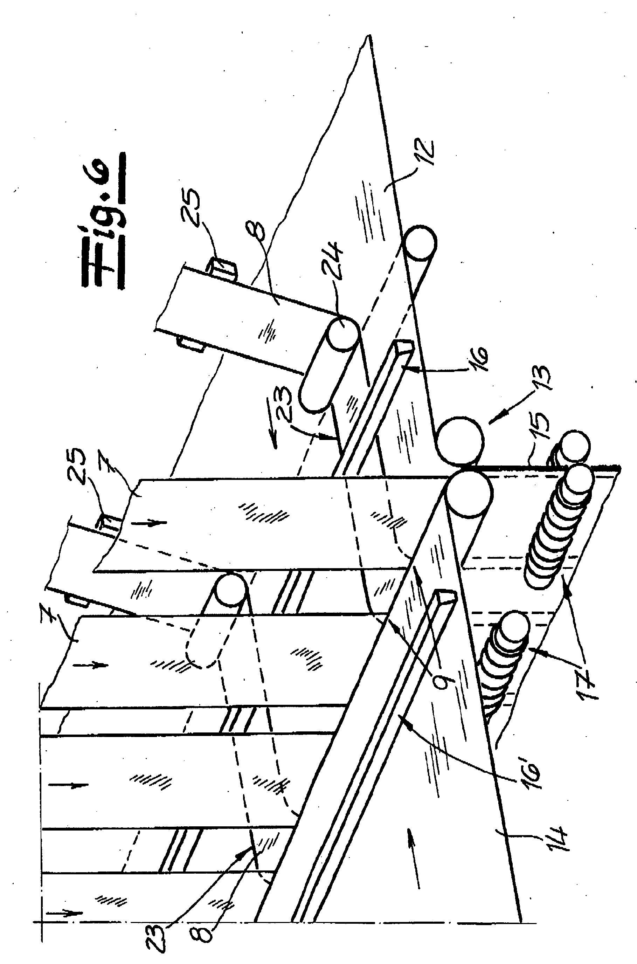

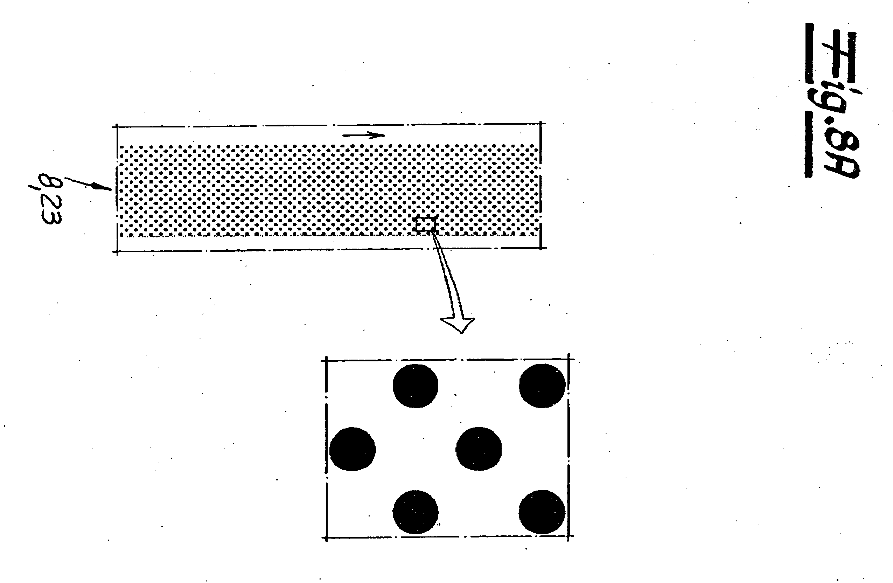

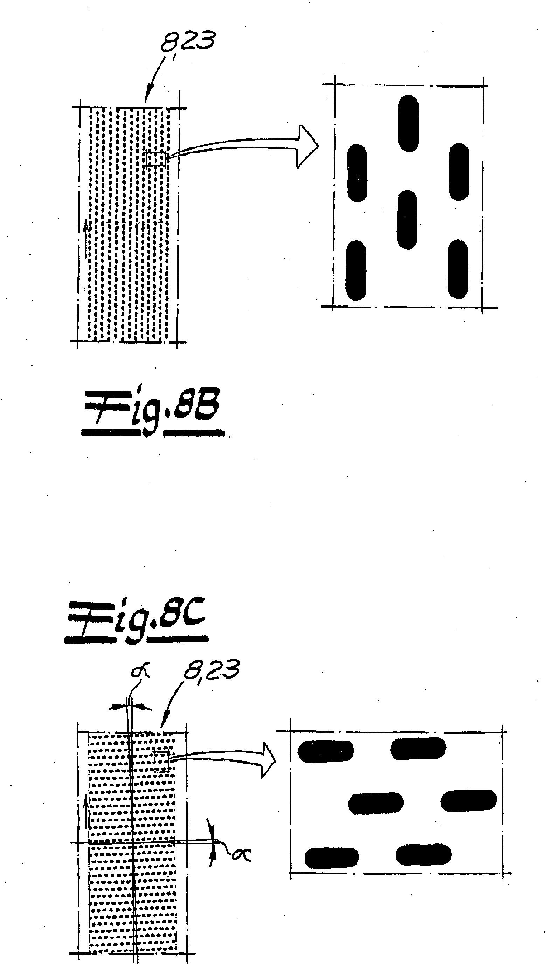

Bei der in Fig. 6 dargestellten Verfahrensvariante werden zur Herstellung der Verstärkungsschichten 8 Verstärkungsstreifen 23 aus einer biegesteifen Polymerfolie oder einem Nonwoven verwendet. Die Verstärkungsstreifen 23 werden mittels einer Beheizungseinrichtung 25 aufgeheizt und mittels Prägewalzen 24 unter Druck und Wärme mit der Nonwovenbahn 12 verpresst. Alternativ kann eine thermische Verbindung auch durch ein Ultraschallschweißen erzeugt werden. Vorteilhafte Muster, die nach einem Aufheizen der Verstärkungsstreifen 13 mittels der Prägewalze 24 oder alternativ durch Ultraschallschweißen erzeugt werden können, sind in den Fig. 8A bis 8C dargestellt. Der in den Fig. 8A bis 8C dargestellte Richtungspfeil zeigt die Längserstreckung der zugeführten Verstärkungsstreifen 23. Die thermische Verbindung erfolgt bevorzugt durch ein gleichmäßiges Muster von punkt-, ellipsen- oder stabförmigen Verbindungsstellen, wobei in Längsrichtung gesehen vorzugsweise ein Versatz zwischen aufeinander folgenden Verbindungsstellen vorgesehen ist. Bei dem in Fig. 8C dargestellten, besonders bevorzugten Muster für eine thermische Verbindung ist auch angedeutet, dass das gleichmäßige Muster aus Verbindungsstellen gegenüber der Längsrichtung leicht, beispielsweise um einen Winkel α von 2°, verkippt sein kann.

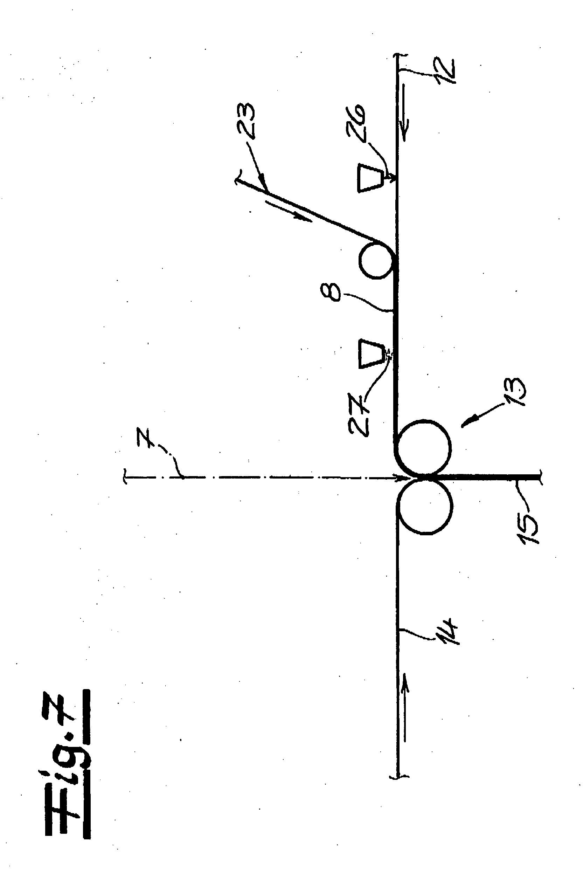

Auch bei dem in Fig. 7 dargestellten Verfahren werden zur Herstellung der Verstärkungsschichten 8 Verstärkungsstreifen 23 aus einer biegesteifen Polymerfolie oder einem Nonwoven verwendet. Die Verstärkungsstreifen 23 werden mit den beidseitigen Nonwovenbahnen 12, 14 verklebt, wobei die Verklebungen so ausgeführt werden, dass die Klebeverbindungen an den beiden Seiten der Verstärkungsstreifen 23 unterschiedliche Haftfestigkeiten aufweisen. Eine gezielte Reduzierung der Haftfestigkeit an zumindest einer Seite der Verstärkungsstreifen 23 wirkt sich positiv auf die Weiterreißfestigkeit des Laminats aus. Bei dem in Fig. 7 dargestellten Verfahren werden die Verstärkungsstreifen 23 zur Erzeugung unterschiedlicher Haftfestigkeiten an einer Seite durch einen flächigen Klebstoffauftrag 26 und an der anderen Seite durch einen partiellen Klebstoffauftrag 27 mit der jeweils angrenzenden Nonwovenbahn 12, 14 verbunden. Hierbei wird vorzugsweise für den partiellen Klebstoffauftrag eine Wirbeldüsentechnik verwendet, die eine großflächige Verteilung des Klebstoffes auf den zu verbindenden Flächen ermöglicht. Die Verstärkungsstreifen 23 können zur Verbesserung der Klebstoffhaftung durch beispielsweise eine Coronabehandlung vorbehandelt werden.

## Patentansprüche

1. Verfahren zur Herstellung einer Materialbahn, aus der Windelverschluss-elemente ausstanzbar sind, die einen elastischen dehnbaren Abschnitt und nichtelastische Anschlussbereiche zur Befestigung an einer Windel sowie zum Anschluss eines Verschlussteils aufweisen, wobei parallele und zueinander beabstandete Folienstreifen (7) aus einem elastisch dehnbaren Polymer zwischen zwei Materialbahnen (12, 14) aus Nonwoven einkaschiert werden, **dadurch gekennzeichnet,**
**dass** biegesteife Verstärkungsschichten (8) aus einem nichtelastischen Polymer in parallelen und zueinander beabstandeten Streifen (11) auf eine erste Nonwovenbahn (12) aufgebracht werden und
**dass** die erste Nonwovenbahn (12) mit den elastischen Folienstreifen (7) und einer zweiten Nonwovenbahn (14) in einem Kaschierwerk (13) zu einem Laminat verbunden wird, welches die Verstärkungsschichten (8) sowie die elastischen Folienstreifen (7) jeweils als Einlage zwischen Außenschichten aus Nonwoven enthält,
wobei die Lage der elastischen Folienstreifen (7) und der Verstärkungsschichten (8) so aufeinander abgestimmt werden, dass die Verstärkungsschichten (8) den Abstand zwischen zwei elastischen Folienstreifen (7) ausfüllen und in beidseitigen Überlappungsbereichen (9) mit den angrenzenden elastischen Folienstreifen (7) verbunden sind.

2. Verfahren nach Anspruch 1, **dadurch gekennzeichnet, dass**, die Verstärkungsschichten (8) durch Extrusionsbeschichtung auf die erste Nonwovenbahn (12) aufgebracht werden.

3. Verfahren nach Anspruch 2, **dadurch gekennzeichnet, dass** auf die erste Nonwovenbahn (12) zumindest im Bereich der aufzubringenden Verstärkungsschichten (8) eine perforierte Folie (21) aufgelegt wird und dass das nichtelastische Polymer für die Verstärkungsschichten (8) durch Extrusionsbeschichtung anschließend auf die perforierte Folie (12) aufgebracht wird, wobei das schmelzeflüssige Polymer die Perforationsöffnungen (22) der Folie (21) durchdringt und sich im Bereich der Perforationsöffnungen (22) mit der Nonwovenbahn (12) verbindet.

4. Verfahren nach Anspruch 2 oder 3, **dadurch gekennzeichnet, dass** zum Auftragen des Polymers ein Auftragswerkzeug verwendet wird, welches an der zu beschichtenden Bahn anliegt, damit das Polymer für die Verstärkungsschichten (8) in die Struktur der Bahn eindringt.

5. Verfahren nach einem der Ansprüche 2 bis 4, **dadurch gekennzeichnet, dass** die erste Nonwovenbahn (12) zur Extrusionsbeschichtung über eine Walze (19) geführt wird, deren Walzenoberfläche eine Struktur mit Erhebungen und/oder Vertiefungen aufweist.

6. Verfahren nach einem der Ansprüche 2 bis 5, **dadurch gekennzeichnet, dass** als Auftragswerkzeug eine Beschichtungsdüse mit einer Mehrzahl von Austrittsöffnungen verwendet wird, wobei die Austrittsöffnungen in einem an den Abstand der Verstärkungsschichten angepassten Abstand angeordnet sind.

7. Verfahren nach einem der Ansprüche 2 bis 5, **dadurch gekennzeichnet, dass** zur Extrusionsbeschichtung mehrere parallel geschaltete Beschichtungsdüsen als Auftragswerkzeuge verwendet werden, deren Abstand vorzugsweise einstellbar ist. ,

8. Verfahren nach Anspruch 1, **dadurch gekennzeichnet, dass** zur Herstellung der Verstärkungsschichten (8) Verstärkungsstreifen (23) aus einer biegesteifen Polymerfolie oder Nonwoven verwendet werden.

9. Verfahren nach Anspruch 8, **dadurch gekennzeichnet, dass** die Verstärkungsstreifien (23) aufgeheizt und in heißem Zustand zumindest mit der ersten Nonwovenbahn (12) verpresst werden.

10. Verfahren nach Anspruch 8 oder 9, **dadurch gekennzeichnet, dass** die Verstärkungsstreifen (23) mittels Prägewalzen (24) unter Druck und Wärme zumindest mit der ersten Nonwovenbahn (12) verpresst werden.

11. Verfahren nach Anspruch 8, **dadurch gekennzeichnet, dass** die Verstärkungsstreifen (23) mit den beidseitigen Nonwovenbahnen (12, 14) verklebt werden, wobei die Verklebungen so ausgeführt werden, dass die Klebeverbindungen an den beiden Seiten der Verstärkungsstreifen (23) unterschiedliche Haftfestigkeiten aufweisen.

12. Verfahren nach Anspruch 11, **dadurch gekennzeichnet, dass** die Verstärkungsstreifen (23) an einer Seite durch einen flächigen Klebstoffauftrag (26) und an der anderen Seite durch einen partiellen Klebstoffauftrag (27) mit der jeweils angrenzenden Nonwovenbahn (12,14) verbunden werden.

13. Verfahren nach Anspruch 12, **dadurch gekennzeichnet, dass** für den partiellen Klebstoffauftrag eine Wirbeldüsentechnik verwendet wird.

14. Verfahren nach einem der Ansprüche 11 bis 13, **dadurch gekennzeichnet, dass** die Verstärkungsstreifen (23) zur Verbesserung der Klebstoffhaftung beidseitig vorbehandelt werden.

15. Verfahren nach Anspruch 11, dass die Verstärkungsstreifen (23) zur Erzeugung unterschiedlicher Klebstoffhaftungen nur an einer Seite vorbehandelt werden.

16. Verfahren nach einem der Ansprüche 1 bis 15, **dadurch gekennzeichnet, dass** die Verstärkungsschichten (8) aus einem Polyolefin, Polyolefin-Copolymer, Styrolpolymer, Cycloolefin-Copolymer, Polyamid, Polyactid, Polyester, thermoplastischem Polyurethan oder Mischungen dieser Polymere hergestellt werden.

17. Verfahren nach einem der Ansprüche 1 bis 16, **dadurch gekennzeichnet, dass** Hot-Melt-Klebstoffe zur Herstellung des Verbundes zwischen den Nonwovenbahnen (12, 14) und den einkaschierten elastischen Folienstreifen (7) verwendet werden.

18. Verfahren nach einem der Ansprüche 1 bis 17, **dadurch gekennzeichnet, dass** die Materialbahn (15) nach dem Kaschierprozess eine Walzenanordnung (17) aus ineinandergreifenden profilierten Streckwalzen durchläuft, in der die Materialbahn (15) im Bereich der einkaschierten elastischen Folienstreifen (7) quer zur Bahnrichtung verstreckt wird.

## Claims

1. Method for fabricating a material web from which nappy
closure elements can be punched, which comprise an elastic stretchable section and non-elastic connecting regions for fastening to a nappy as well as for connection of a closure part, wherein parallel and spaced-apart film strips (7) of an elastically stretchable polymer are laminated between two material webs (12, 14) of nonwoven, **characterised in**
**that** bend-resistant reinforcing layers (8) of a non-elastic polymer are applied in parallel and spaced-apart strips (11) to a first nonwoven web (12) and
**that** the first nonwoven web (12) is joined to the elastic film strip (7) and a second nonwoven web (14) in a laminating apparatus (13) to form a laminate which contains the reinforcing layers (8) as well as the elastic film strips (7) in each case as an insert between outer layers of nonwoven,
wherein the position of the elastic film strips (7) and the reinforcing layers (8) are matched to one another so that the reinforcing layers (8) fill the spacing between two elastic film strips (7) and are connected to the adjacent film strips (7) in overlap regions (9) on both sides.

2. The method according to claim 1, **characterised in that** the reinforcing layers (8) are applied to the first nonwoven web (12) by extrusion coating.

3. The method according to claim 2, **characterised in that** a perforated film (21) is applied to the first nonwoven web (12) at least in the region of the reinforcing layers (8) to be applied and that the elastic polymer for the reinforcing layers (8) is then applied to the perforated film (12) by extrusion coating, wherein the fusible polymer passes through the perforation openings (22) of the film (21) and joins to the nonwoven web (12) in the region of the perforation openings (22).

4. The method according to claim 2 or 3, **characterised in that** an application tool is used for application of the polymer, which bears against the web to be coated so that the polymer for the reinforcing layers (8) penetrates into the structure of the web.

5. The method according to any one of claims 2 to 4, **characterised in that** the first nonwoven web (12) for the extrusion coating is guided over a roller (19) whose roller surface has a structure comprising elevations and/or recesses.

6. The method according to any one of claims 2 to 5, **characterised in that** a coating nozzle having a plurality of outlet openings is used as an application tool, wherein the outlet openings are disposed at a distance adapted to the spacing of the reinforcing layers.

7. The method according to any one of claims 2 to 5, **characterised in that** a plurality of parallel-connected coating nozzles are used as application tools for the extrusion coating, the spacing whereof is preferably adjustable.

8. The method according to claim 1, **characterised in that** reinforcing strips (23) of a bend-resistant polymer film or nonwoven are used to fabricate the reinforcing layers (8).

9. The method according to claim 8, **characterised in that** the reinforcing strips (23) are heated and pressed in the hot state at least to the first nonwoven web (12).

10. The method according to claim 8 or 9, **characterised in that** the reinforcing strips (23) are pressed by means of embossing rollers (24) under pressure and heat at least with the first nonwoven web (12).

11. The method according to claim 8, **characterised in that** the reinforcing strips (23) are adhesively bonded to the bilateral nonwoven webs (12, 14), wherein the adhesive bonds are executed so that the adhesive connections have different adhesive strengths on both sides of the reinforcing strips (23).

12. The method according to claim 11, **characterised in that** the reinforcing strips (23) are joined to the respectively adjacent nonwoven web (12, 14) on one side by an extensive adhesive application (26) and on the other side by a partial adhesive application (27).

13. The method according to claim 12, **characterised in that** a swirl nozzle technique is used for the partial adhesive application.

14. The method according to any one of claims 11 to 13, **characterised in that** the reinforcing strips (23) are pre-treated on both sides to improve the adhesion.

15. The method according to claim 11, **characterised in that** the reinforcing strips (23) are pre-treated only on one side to produce different adhesions.

16. The method according to any one of claims 1 to 15, **characterised in that** the reinforcing layers (8) are made of a polyolefin, polyolefin copolymer, styrene polymer, cycloolefin copolymer, polyamide, polylactide, polyester, thermoplastic polyurethane or mixtures of these polymers.

17. The method according to any one of claims 1 to 16, **characterised in that** hot-melt adhesives are used to produce the bond between the nonwoven webs (12, 14) and the laminated elastic film strips (7).

18. The method according to any one of claims 1 to 17, **characterised in that** after the laminating process the material web (15) runs through a roller arrangement (17) of intermeshing profiled stretch rollers in which the material web (15) is stretched transversely to the web direction in the region of the laminated elastic film strips (7).

## Revendications

1. Procédé de fabrication d'une bande de matériau dans laquelle peuvent être découpés des éléments de fermeture de couche qui présentent une section élastiquement extensible et des zones de raccordement non élastiques pour la fixation à une couche et pour le raccordement d'une pièce de fermeture, des bandes de film parallèles et espacées entre elles (7) en polymère élastiquement extensible étant plaquées entre deux bandes de matériau (12, 14) en non-tissé, **caractérisé en ce que**
des couches de renfort rigides à la flexion (8) en polymère non élastique sont appliquées dans des bandes (11) parallèles et espacées entre elles sur une première bande de non-tissé (12) et
que la première bande de non-tissé (12) est reliée aux bandes de film élastique (7) et à une seconde bande de non-tissé (14) dans une unité de placage (13) pour former un laminé qui contient les couches de renfort (8) et les bandes de film non tissé (7) respectivement sous la forme d'une garniture placée entre les couches extérieures en non-tissé,
la couche de bandes de film élastique (7) et les couches de renfort (8) étant adaptées mutuellement de manière à ce que les couches de renfort (8) comblent la distance entre deux bandes de film élastique (7) et soient reliées dans les deux zones de chevauchement bilatérales (9) aux bandes de film élastique adjacentes (7).

2. Procédé selon la revendication 1, **caractérisé en ce que** les couches de renfort (8) sont appliquées sur la première bande de non-tissé (12) par revêtement par extrusion.

3. Procédé selon la revendication 2, **caractérisé en ce qu'**un film perforé (21) est posé sur la première bande de non-tissé (12) du moins au niveau des couches de renfort (8) à appliquer et que le polymère non élastique destiné aux couches de renfort (8) est ensuite appliqué sur le film perforé (21) par revêtement par extrusion, le polymère liquide en fusion traversant les orifices perforés (22) du film (21) et se joignant à la bande de non-tissé (12) au niveau des orifices perforés (22).

4. Procédé selon la revendication 2 ou 3, **caractérisé en ce que**, pour appliquer le polymère, on utilise un outil d'application qui repose sur la bande à revêtir afin que le polymère destiné aux couches de renfort (8) pénètre dans la structure de la bande.

5. Procédé selon une des revendications 2 à 4, **caractérisé en ce que** la première bande de non-tissé (12) est guidée pour revêtement par extrusion via un rouleau (19) dont la surface de rouleau présente une structure à aspérités et/ou creux.

6. Procédé selon une des revendications 2 à 5, **caractérisé en ce qu'**on utilise comme outil d'application une buse de revêtement dotée d'une pluralité d'orifices de sortie, les orifices de sortie étant disposés avec un espacement adapté à l'espacement des couches de renfort.

7. Procédé selon une des revendications 2 à 5, **caractérisé en ce qu'**on utilise pour le revêtement par extrusion plusieurs buses de revêtement branchées parallèlement faisant office d'outils d'application et dont l'espacement est de préférence réglable.

8. Procédé selon la revendication 1, **caractérisé en ce que**, pour réaliser les couches de renfort (8), on utilise des bandes de renfort (23) en film de polymère ou non-tissé rigide à la flexion.

9. Procédé selon la revendication 8, **caractérisé en ce que** les bandes de renfort (23) sont chauffées et comprimées à l'état chaud du moins avec la première bande de non-tissé (12).

10. Procédé selon la revendication 8 ou 9, **caractérisé en ce que** les bandes de renfort (23) sont comprimées au moyen de rouleaux de compression (24) sous pression et chaleur au moins avec la première bande de non-tissé (12).

11. Procédé selon la revendication 8, **caractérisé en ce que** les bandes de renfort (23) sont collées aux bandes de non-tissé bilatérales (12, 14), les collages étant réalisés de manière à ce que les raccords collés présentent des adhérences différentes sur les deux faces des bandes de renfort (23).

12. Procédé selon la revendication 11, **caractérisé en ce que** les bandes de renfort (23) sont raccordées par une face par application de colle (26) sur toute leur surface et par l'autre face par application partielle de colle (27) à la bande de non-tissé respectivement adjacente (12, 14).

13. Procédé selon la revendication 12, **caractérisé en ce qu'**on utilise une technique de buse tourbillonnante pour l'application partielle de colle.

14. Procédé selon une des revendications 11 à 13, **caractérisé en ce que** les bandes de renfort (23) sont prétraitées sur leurs deux faces pour améliorer l'adhérence de la colle.

15. Procédé selon la revendication 11, **caractérisé en ce que** les bandes de renfort (23) sont prétraitées seulement sur une face pour créer des adhérences variables de la colle.

16. Procédé selon une des revendications 1 à 15, **caractérisé en ce que** les couches de renfort (8) sont faites d'un mélange de polyoléfine, copolymère de polyoléfine, polymère de styrène, copolymère de cyclooléfine, polyamide, polyactide, polyester, polyuréthane thermoplastique ou mélanges de ces polymères.

17. Procédé selon une des revendications 1 à 16, **caractérisé en ce que** des colles thermofusibles sont utilisées pour fabriquer le composite entre les bandes de non-tissé (12, 14) et les bandes de film élastiques plaquées (7).

18. Procédé selon une des revendications 1 à 17, **caractérisé en ce que** la bande de matériau (15) passe après le processus de placage dans un dispositif à rouleaux (17) fait de rouleaux d'étirage profilés s'engrenant les uns dans les autres et dans laquelle la bande de matériau (15) est étirée au niveau des bandes de film élastiques plaquées (7) transversalement au sens de la bande.
